# EUROPÄISCHE PATENTSCHRIFT

(11) **EP 2 751 100 B1**
(45) Veröffentlichungstag und Bekanntmachung des Hinweises auf die Patenterteilung: **14.10.2015**
(21) Anmeldenummer: 12753705.8
(22) Anmeldetag: 27.08.2012
(51) Int. Cl.: C07D 417/04, C07D 417/06

(54) **VERFAHREN ZUM HERSTELLEN VON [3-[(6-CHLOR-3-PRIDINYL)METHYL]-2-THIAZOLIDINYLIDEN]CYANAMID**
METHOD FOR PRODUCING [3-[(6-CHLOR-3-PRIDINYL)METHYL]-2-THIAZOLIDINYLIDENE]CYANAMIDE
PROCÉDÉ DE PRÉPARATION DE [3-[(6-CHLORO-3-PYRIDINYL)MÉTHYL]-2-THIAZOLIDINYLIDÈNE]-CYANAMIDE

(30) Priorität: 02.09.2011 EP 11179883
(43) Veröffentlichungstag der Anmeldung: 09.07.2014
(73) Patentinhaber: Bayer Intellectual Property GmbH, 40789 Monheim am Rhein (DE)
(72) Erfinder: LUI, Norbert, 51519 Odenthal (DE); ANTONS, Stefan, 51373 Leverkusen (DE); HEINRICH, Jens-Dietmar, 51381 Leverkusen (DE)
(74) Vertreter: BIP Patents
(86) Internationale Anmeldenummer: PCT/EP2012/066602
(87) Internationale Veröffentlichungsnummer: WO 2013/030152

(56) Entgegenhaltungen:
- EP-A1- 1 024 140
- JESCHKE, P. ET AL: "Thiacloprid (Bay YRC 2894) - a new member of the chloronicotinyl insecticide (CNI) family", PFLANZENSCHUTZ-NACHRICHTEN BAYER (ENGLISH EDITION), CODEN: PNBED6; ISSN: 0170-0405, Bd. 54, Nr. 2, 2001, Seiten 147-160, XP002684766,

## Beschreibung

Die vorliegende Erfindung betrifft ein Verfahren zum Herstellen von [3-[(6-Chlor-3-pridinyl)methyl]-2-thiazolidinyliden] cyanamid der Formel (IV), einem Pflanzenschutzwirkstoff.

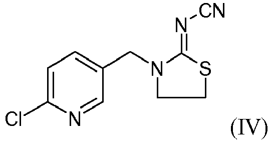

Die Herstellung erfolgt in mehreren isolierten Schritten, ausgehend von Cyanimino-1,3-thiazolidin und 5-Chlormethyl-2-chlorpyridin (EP 1 024 140), N-(2-Chlor-5-pyridylmethyl)cysteamin und Dimethylcyanodithioimidocarbonat (EP 0 235 725). In diesen Fällen muss zuerst das Cyanimino-1,3-thiazolidin oder das N-(2-Chlor-5-pyridylmethyl)cysteamin hergestellt und gereinigt werden und ggf. noch das Endprodukt gereinigt werden. Zudem ist die Gesamtausbeute über alle Stufen ausgehend von Dimethyl-N-cyanocarbonimidodithiocarbonat und Cysteamin unbefriedigend.

Im Hinblick auf die vorstehend geschilderten Nachteile und Probleme besteht der Bedarf ein Verfahren bereitzustellen, das ausgehend von Dimethyl-N-cyanocarbonimidodithio-carbonat das [3-[(6-Chlor-3-pridinyl)methyl]-2-thiazolidinyliden] cyanamid ohne Isolierung von Zwischenstufen mit hohen Ausbeuten und hoher Selektivität zugänglich macht.

Gelöst wird diese Aufgabe durch das folgende Verfahren:
Cysteamin oder ein Salz hiervon (Formel (I)) 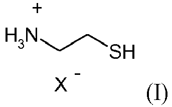
und Dimethyl-N-cyanocarbonimidodithiocarbonat der Formel (II) 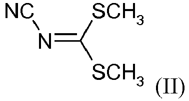
werden in Gegenwart von Wasser und gegebenenfalls unter Zusatz eines Lösungsmittels und einer Base umgesetzt und ohne Zwischenisolierung von Cyanimino-1,3-thiazolidin mit 2-Chlor-5-chlormethylpyridin der Formel (III) 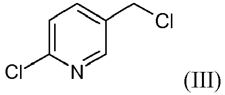
alkyliert. X steht hierbei für einen Säurerest wie beispielsweise Halogen, Acetat, Sulfat oder Hydrogensulfat.

Die Reaktion erfolgt nach dem Schema 1,

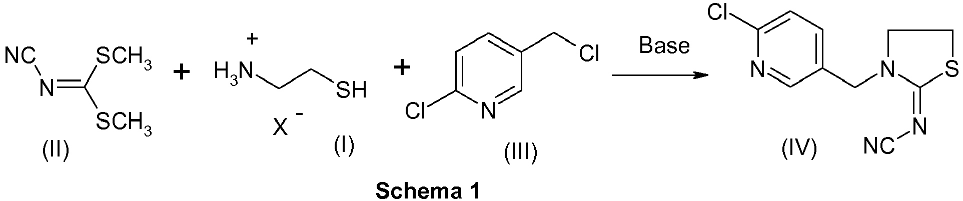

Es ist überraschend, dass ohne Zwischenisolierung des Cyanimino-1,3-thiazolidin eine höhere Ausbeute erzielt wird als bei der Isolierung des Zwischenproduktes, ohne dass die sehr gute Qualität des Zielproduktes verschlechtert wird.

Die Anmeldung betrifft demzufolge ein Verfahren zur Herstellung von [3-[(6-Chlor-3-pridinyl)methyl]-2-thiazolidinyliden] cyanamid, umfassend die folgenden Schritte:
(i) Reaktion von Dimethyl-N-cyanocarbonimidodithiocarbonat und Cysteamin oder ein Salz davon in Gegenwart einer Base;
(ii) Umsetzen der Reaktionsmischung mit 5-Chlormethyl-2-chlorpyridin;

Bevorzugt ist die Durchführung des erfindungsgemäßen Verfahrens unter Verwendung von Verbindungen der Formel (I), in welchen X für einen Säurerest wie beispielsweise Halogen, Acetat, Sulfat oder Hydrogensulfat steht.

Bevorzugt steht X für Chlorid, Sulfat oder Hydrogensulfat.

Für das erfindungsgemäße Verfahren werden als Basen Alkalimetall- und Erdalkalimetallhydroxide, Alkalimetallcarbonate oder Alkalimetall-hydrogencarbonate eingesetzt. Vorzugsweise werden Natriumhydroxid, Kaliumhydroxid, Natriumhydrogencarbonat, Kalium-hydrogencarbonat, Natriumcarbonat und Kaliumcarbonat eingesetzt.

Die bei dem erfindungsgemäßen Verfahren als Ausgangsstoffe zu verwendenden Cysteaminsalze der Formel (I) sind kommerziell erhältlich und allgemein bekannte Verbindungen der organischen Chemie.

Das erfindungsgemäße Verfahren läuft in Anwesenheit von Wasser ab. Es können auch wasserhaltige Lösungsmittelgemische verwendet werden. Diese können neben Wasser auch andere Lösungsmittel enthalten. Als Beispiele sind zu nennen Alkohole wie Methanol, Ethanol, Isopropanol, n-Butanol, isoButanol; Ether, wie Ethylpropylether, Methyl-tert-butylether, n-Butylether, Anisol, Phenetol, Cyclohexylmethylether, Dimethylether, Diethylether, Dimethylglycol Diphenylether, Dipropylether, Diisopropylether, Di-n-butylether, Diisobutylether, Diisoamylether, Ethylenglycoldimethylether, Isopropylethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methylmorpholin, Pyridin und alkylierte Pyridine; Nitrile wie Acetonitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan; beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidon, N-Methylcaprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin; Ketone wie Aceton, Acetophenon, Methylethylketon, Methylbutylketon.

Bevorzugte Cosolventien sind: Methanol, Ethanol, THF, n-Butanol.

In einer besonders bevorzugten Ausführungsform läuft die Reaktion in einer Mischung von Wasser und n-Butanol ab.

Als Katalysatoren können Phasentransferkatalysatoren wie z.B. quartäre Ammoniumsalze eingesetzt werden.

Außerdem kann das erfindungsgemäße Verfahren in wässrigen Zweiphasensystemen ablaufen. Dabei wird ein weiteres nicht- oder nur sehr begrenzt wassermischbares Lösungsmittel verwendet.

Im Schritt (i) wird das Cysteamin-Hydrochlorid oder das Cysteamin in einer Lösung von Alkalimetallhydroxiden, Alkalimetallcarbonaten oder Alkalimetallhydrogencarbonaten gelöst. Als Lösungsmittel kann Wasser oder ein Gemisch mit Wasser verwendet werden. Dieser Vorgang kann bei Raumtemperatur erfolgen. Anschließend wird die Lösung auf eine Temperatur von 5 bis 40°C, bevorzugt auf 10 bis 20°C abgekühlt. Das Dimethyl-N-cyanocarbonimidodithiocarbonat wird (ggf als Schmelze ) zudosiert.

Das molare Verhältnis von Cysteamin-Hydrochlorid zu Dimethyl-N-cyanocarbonimidodithiocarbonat kann von 1 : 0,7 bis 1 : 1,5 betragen, bevorzugt 1 : 0,95 bis 1 : 1,05.

Nach Beendigung der Dosierung wird noch 0,5 bis 10 Stunden bei Temperaturen von 10 bis 25 °C, bevorzugt 1 bis 4 Stunden nachgerührt. Längere Reaktionszeiten sind unkritisch.

Die Reaktion wird zweckmäßigerweise unter Atmosphärendruck durchgeführt, jedoch kann auch unter vermindertem oder erhöhtem Druck gearbeitet werden.

Im Schritt (ii) kann weitere Base in Form von Alkalimetallhydroxiden, Alkalimetallcarbonaten oder Alkalimetallhydrogencarbonaten zugesetzt werden. Der Zusatz von 2-Chlor-5-chlormethylpyridin erfolgt in gelöster Form, z.B. in n-Butanol, oder als Schmelze. Das Verfahren im Schritt (ii) kann in einem weiten Temperaturbereich durchgeführt werden, beispielsweise zwischen 30°C und 100°C, vorzugsweise zwischen 50°C und 80°C.

Die Reaktion wird zweckmäßigerweise unter Atmosphärendruck durchgeführt, jedoch kann auch unter vermindertem oder erhöhtem Druck gearbeitet werden.

Bei der praktischen Durchführung des Verfahrens wird beispielsweise 1 Mol der Verbindung (I) mit 1 Mol der Formel (II) und mit 1,05 Mol der Formel (III) umgesetzt.

Das erfindungsgemäße Verfahren lässt sich diskontinuierlich oder kontinuierlich durchführen. Das Verfahren lässt sich ferner unter Normaldruck, Unter- oder Überdruck durchführen.

Die Aufarbeitung kann durch Filtration oder Extraktion erfolgen.

Das erfindungsgemäße Verfahren zum Herstellen von [3-[(6-Chlor-3-pridinyl)methyl]-2-thiazolidinyliden] cyanamid wird in den nachstehenden Beispielen beschrieben, welche die obige Beschreibung weiter illustrieren.

### Herstellungsbeispiele:

### Beispiel 1

13,7g Natriumcarbonat (0,129 Mol) werden in 78g Wasser gelöst. Bei RT werden 15,0g Cysteaminhydrochlorid 98%ig (0,129 Mol) zugegeben. Die Suspension wird auf 10°C abgekühlt und portionsweise mit 21,0g Cyanimidodithiokohlensäuredimethylester 90%ig (0,129 Mol) versetzt. Es ist sofort eine leichte Gasentwicklung zu erkennen. Anschließend lässt man innerhalb von 2h die Temperatur auf 20°C kommen. HPLC Kontrolle zeigt vollständigen Umsatz.

Es werden 130ml n-Butanol hinzugegeben. Die Suspension wird unter vermindertem Druck bei max. 50°C am Wasserabscheider vom Wasser befreit. Anschließend wird die Suspension nach Belüften mit 39,3g Kaliumcarbonat (0,285 Mol) versetzt und auf 75°C erwärmt. Es wird 1h nachgerührt.

Innerhalb von 2h wird eine Lösung von 22,1g (0,136 Mol) 2-Chlor-5-chlormethylpyridin in 44,1g n-Butanol zugetropft. Anschließend wird auf 80°C erwärmt und 2h bei dieser Temperatur nachgerührt. Nach Abkühlen auf 70°C werden 130ml Wasser von 70°C zugesetzt und bei 70°C die Phasen getrennt. Die wässrige Phase wird bei 70°C zweimal mit je 20ml n-Butanol extrahiert. Die vereinigten Butanolphasen werden auf 25°C abgekühlt. Der ausgefallene Feststoff wird abgesaugt. Die Mutterlauge wurde auf -5°C abgekühlt. Der ausgefallene Feststoff wird über den ersten Feststoff abgesaugt und einmal mit 20ml -10°C kaltem n-Butanol gewaschen. Der erhaltene Feststoff wird im Vakuumtrockenschrank bei 40°C getrocknet.

Man erhält 26,5 g weißen Feststoff mit einer Reinheit von 98,2% (entspricht 79,6% d. Th.).

¹H-NMR, DMSO, 298K: 3,50ppm (t) 2H; 3,91ppm (t) 2H; 4,64ppm (s) 2H; 7,54ppm (d) 1H; 7,79ppm (dd) 1H; 8,38ppm (d) 1H.

## Patentansprüche

1. Verfahren zur Herstellung von [3-[(6-Chlor-3-pridinyl)methyl]-2-thiazolidinyliden] cyanamid, umfassend die folgenden Schritte:
(i) Reaktion von Dimethyl-N-cyanocarbonimidodithiocarbonat und Cysteamin oder ein Salz davon in Gegenwart einer Base;
(ii) Umsetzen der Reaktionsmischung mit 5-Chlormethyl-2-chlorpyridin;

2. Verfahren gemäß Anspruch 1, bei dem das Cysteamin als Salz der Formel (I) eingesetzt wird 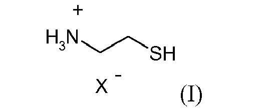 und X⁻ ausgewählt ist aus Chlorid, Sulfat oder Hydrogensulfat.

3. Verfahren gemäß einem der Ansprüche 1 oder 2 in Anwesenheit einer der folgenden Basen: Natriumhydroxid, Kaliumhydroxid, Natriumhydrogencarbonat, Kaliumhydrogencarbonat, Natriumcarbonat oder Kaliumcarbonat.

4. Verfahren gemäß einem der Ansprüche 1 bis 3, wobei die Reaktion in einem Gemisch aus Wasser und n-Butanol durchgeführt wird.

## Claims

1. Process for preparing [3-[(6-chloro-3-pyridinyl)methyl]-2-thiazolidinylidene]cyanamide, comprising the following steps:
(i) reaction of dimethyl N-cyanocarbonimidodithiocarbonate and cysteamine or a salt thereof in the presence of a base;
(ii) reaction of the reaction mixture with 5-chloromethyl-2-chloropyridine.

2. Process according to Claim 1, in which the cysteamine is used as the salt of the formula (I) 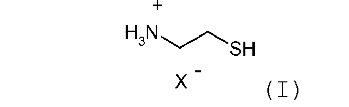 and X⁻ is selected from chloride, sulphate and hydrogensulphate.

3. Process according to either of Claims 1 and 2, in the presence of one of the following bases: sodium hydroxide, potassium hydroxide, sodium hydrogencarbonate, potassium hydrogencarbonate, sodium carbonate and potassium carbonate.

4. Process according to any of Claims 1 to 3, wherein the reaction is performed in a mixture of water and n-butanol.

## Revendications

1. Procédé pour la préparation de cyanamide de [3-[(6-chloro-3-pyridinyl)méthyl]-2-thiazolidinylidène] comprenant les étapes suivantes :
(i) réaction de diméthyl-N-cyanocarbonimidodithiocarbonate et de cystéamine ou d'un sel de celle-ci en présence d'une base ;
(ii) transformation du mélange réactionnel avec de la 5-chlorométhyl-2-chloropyridine.

2. Procédé selon la revendication 1, dans lequel la cystéamine est utilisée sous forme de sel de formule (I) 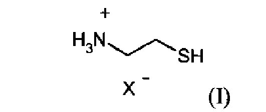 et X⁻ est choisi parmi le chlorure, le sulfate ou l'hydrogénosulfate.

3. Procédé selon l'une quelconque des revendications 1 ou 2 en présence d'une des bases suivantes : hydroxyde de sodium, hydroxyde de potassium, hydrogénocarbonate de sodium, hydrogénocarbonate de potassium, carbonate de sodium ou carbonate de potassium.

4. Procédé selon l'une quelconque des revendications 1 à 3, la réaction étant réalisée dans un mélange d'eau et de n-butanol.
